# EUROPEAN PATENT SPECIFICATION

(11) **EP 4 087 634 B1**
(45) Date of publication and mention of the grant of the patent: **15.04.2026**
(21) Application number: 20912317.3
(22) Date of filing: 22.12.2020
(51) Int. Cl.: A61M 16/08, A61M 39/10, A62B 9/04

(54) **CONNECTORS FOR RESPIRATORY GASES TUBES**
VERBINDER FÜR ATEMGASROHRE
RACCORDS POUR TUBES DE GAZ RESPIRATOIRES

(30) Priority: 10.01.2020 US 202062959734 P
(43) Date of publication of application: 16.11.2022
(73) Proprietor: Fisher & Paykel Healthcare Limited, East Tamaki Auckland 2013 (NZ)
(72) Inventor: RANJITSINGH, Michael, Auckland, 1010 (NZ); MCCOOL, Kiel Anthony, Auckland, 1010 (NZ); MILLAR, Gavin Walsh, Auckland, 1010 (NZ); LARA GARCIA, Abel, Auckland, 1010 (NZ)
(74) Representative: Gill Jennings & Every LLP
(86) International application number: PCT/IB2020/062313
(87) International publication number: WO 2021/140399

(56) References cited:
- EP-B1- 2 469 146
- WO-A1-2004/108218
- WO-A1-2010/067218
- WO-A1-2012/018362
- WO-A1-2013/022356
- WO-A1-95/30448
- WO-A2-2011/094018
- WO-A2-2019/222701
- DE-A1- 3 709 122
- JP-A- 2005 000 466
- US-A- 5 057 093
- US-A- 5 873 361
- US-A1- 2008 172 039
- US-A1- 2008 284 167
- US-A1- 2012 123 392
- US-A1- 2012 136 272
- US-A1- 2017 120 033
- US-A1- 2017 296 095
- US-A1- 2018 339 132
- US-B1- 6 581 593
- US-B1- 6 851 427
- US-B1- 7 669 595
- US-B1- 7 669 595

## Description

### FIELD OF THE INVENTION

The present invention relates to connectors for medical use, and particularly respiratory gases tube assemblies and connectors for use in breathing circuits suitable for delivering humidified gases to or from a patient, such as in respiratory humidification systems.

### BACKGROUND

In breathing circuits, various components transport warm and/or humidified gases to and from patients. Respiratory humidification helps reduce the likelihood of infection and/or tissue damage.

Humidifiers are used to provide humidification to the gases. Respiratory gases tubes are used to transport the humidified gases to and from a patient and to connect together any devices as part of a breathing circuit.

A breathing circuit may provide for a complete circuit of breathing gases to and from the patient. In some cases, no tube is provided to remove gases from the patient and gas can be exhaled directly to atmosphere. In other cases, a full breathing circuit is provided to deliver gases to a patient as well as to remove them.
WO 95/30448 A1 discusses an endotracheal catheter assembly and method for deep intrapulmonary, aerosol delivery of liquid drugs and other liquid therapeutic agents. US 7 669 595 B1 discusses a junction device for administering nebulizer treatments directly to a supply of oxygen supplied to a patient mask which includes a supply conduit being couplable to an oxygen supply and a patient mask to allow oxygen from the oxygen supply to be supplied to the patient mask.

### SUMMARY

Disclosed is a respiratory gases assembly and/or connector and/or connector assembly, for use with a respiratory apparatus and a breathing circuit.

In a first aspect there may be provided a connector assembly according to appended claim 1.

In some embodiments, the accessory is integrally formed with the connector elbow or wherein the accessory is disconnectable and connectable with the accessory end.

In some embodiments, the connector elbow is an accessory tube connector elbow, the accessory is an accessory tube, and the accessory end is an accessory tube end configured to connect with the accessory tube, and optionally wherein the accessory tube connector elbow comprises a lumen extending from the port end to the accessory end.

In some embodiments, the accessory tube is one or more of:
a pressure line,
a sampling tube.

In some embodiments, the connector elbow comprises a barbed connection at the accessory end configured connect with the accessory tube.

In some embodiments, the connector elbow comprises a frictional connection at the accessory end configured connect with the accessory tube.

In some embodiments, the connector elbow is a sensor connector elbow, the accessory is a sensor lead, and the accessory end is a sensor lead end configured to connect with the sensor lead, and wherein the sensor connector elbow comprises a sensor.

In some embodiments, a central axis of the accessory end of the connector elbow is rotatable relative to the port connector to align a central axis of the lumen of the port connector.

In some embodiments, the port connector comprises one or more protrusions located within the lumen of the port connector.

In some embodiments, the one or more protrusions extend in a direction along the lumen of the port connector.

In some embodiments, the one or more protrusions are arranged equidistantly around the lumen of the port connector.

In some embodiments, the one or more protrusions comprises three protrusions arranged equidistantly around the lumen of the port connector.

In some embodiments, the connection mechanism is located around at least a part of a perimeter of the port.

In some embodiments, the port connector is or comprises a wye piece.

In some embodiments, the port connector is a pressure port connector, and/or a sampling tube connector, optionally the port connector is an accessory port connector.

In some embodiments, the connection mechanism of the port is one or more of:
a thread
a bayonet.

In some embodiments, the port is substantially cylindrical.

In some embodiments, the port sealing surface is located on an internal surface of the port.

In some embodiments, the connector elbow sealing surface is located on an external surface of the connector elbow.

In some embodiments, the port sealing surface is located on an external surface of the port.

In some embodiments, the connector elbow sealing surface is located on an internal surface of the connector elbow.

In some embodiments, the port sealing surface is located on an end surface of the port.

In some embodiments, the connector elbow sealing surface is located on an end surface of the connector elbow.

In some embodiments, the port comprises a distal portion located at a connector elbow connection end, the distal portion comprising an distal portion tapering surface.

In some embodiments, the distal portion tapering surface tapers towards a proximal end of the port.

In some embodiments, the distal portion provides for alignment of the connector elbow and the port when the connector elbow is brought into engagement with the port.

In some embodiments, the port comprises a sealing portion, the sealing portion comprising a sealing portion tapering surface.

In some embodiments, the sealing portion tapering surface tapers towards a proximal end of the port.

In some embodiments, the sealing portion comprises the port sealing surface.

In some embodiments, the port comprises a proximal portion, the proximal portion located at a proximal end of the port.

In some embodiments, the port comprises an intermediate portion located between the distal portion and the sealing portion.

In some embodiments, the intermediate portion is configured to maintain alignment of the connector elbow and the port, when the connector elbow and the port are engaged.

In some embodiments, the port end of the connector elbow comprises a connector elbow tapering surface.

In some embodiments, the connector elbow tapering surface comprises the connector elbow sealing surface.

In some embodiments, the port extends in a direction away from the lumen of the port connector.

In some embodiments, the port extends in a direction substantially perpendicular to a central axis of the lumen of the port connector.

In some embodiments, the connector elbow has an angle between the accessory end and the port end of about 10 degrees to about 120 degrees, or about 45 to about 115 degrees, or about 80 to about 100 degrees, or about 90 degrees.

In some embodiments, the collar comprises a corresponding connection mechanism on an internal surface of the collar to engage with the connection mechanism of the port connector.

In some embodiments, the collar is rotatable relative to the connector elbow.

In some embodiments, the connector elbow comprises at least one protrusion extending outwardly from an external surface of the connector elbow, the at least one protrusion configured to retain the collar on the connector elbow.

In some embodiments, the protrusion extends circumferentially about at least part of the connector elbow.

In some embodiments, the protrusion is located near a port end of the connector elbow.

In some embodiments, the collar comprises a protrusion, the protrusion configured to engage with the protrusion of the connector elbow.

In another aspect there may be provided a connector assembly, the connector assembly comprising:
a port providing for a passageway into a lumen of the port connector,
an connector elbow, the connector elbow comprising:
   an accessory end configured to connect with an accessory,
      and
   a port end configured to connect with the port,
wherein in a first configuration the connector elbow is engaged with the port connector but able to rotate relative to the port connector to align a central axis of the accessory end with a central axis of the lumen of the port connector, and
wherein:
   a) in a second configuration, after alignment of the central axis of the accessory end of the connector elbow and the central axis of the lumen of the port connector, the connector elbow is prevented from rotating relative to the port connector to maintain alignment of the accessory and the lumen of the port connector, or
   b) in a second configuration, after alignment of the central axis of the accessory end of the connector elbow and the central axis of the lumen of the port connector, the resistance to relative rotation of the elbow connector and the port connector is increased to maintain alignment of the accessory and the lumen of the port connector.

In some embodiments, the connector elbow is an accessory tube connector elbow, the accessory is an accessory tube, and the accessory end is an accessory tube end configured to connect with the accessory tube.

In some embodiments, the connector elbow is an accessory tube connector elbow comprising a lumen extending from the port end to the accessory connector end.

In some embodiments, the accessory tube is one or more of:
a pressure line
a sampling tube.

In some embodiments, the connector elbow is a sensor connector elbow, the accessory is a sensor lead, and the accessory end is a sensor lead end configured to connect with the sensor lead, and wherein the sensor connector elbow comprises a sensor.

In some embodiments, the connector elbow comprises a first connection mechanism and the port connector comprise a second connection mechanism.

In some embodiments, the first connection mechanism and/or the second connection mechanism are threaded connections.

In some embodiments, the first connection mechanism and second connection mechanism urges an connector elbow sealing surface connector elbow into engagement with a port sealing surface.

In some embodiments, engaging the first connection mechanism and second connection mechanism, in a first connector configuration, provides for the first configuration.

In some embodiments, engaging the first connection mechanism and second connection mechanism, in a second connector configuration, provides for the second configuration.

In another aspect there may be provided a respiratory gases tube assembly, the respiratory gases tube assembly comprising:
a respiratory gases tube configured to transport a breathing gas, the respiratory gases tube comprising a lumen extending from a first end of the respiratory gases tube to a second end of the respiratory gases tube, and
a connector assembly according to any one of the aspects described above.

In some embodiments, the port connector is integrally formed with the respiratory gases tube.

In some embodiments, the port connector is provided as a separate connector component which is connectable with the respiratory gases tube.

In some embodiments, the respiratory gases tube is or forms part of an expiratory limb.

In some embodiments, the respiratory gases tube is or forms part of an inspiratory limb.

In some embodiments, the port extends in a direction substantially perpendicular to a respiratory gases tube

In another aspect there may be provided a respiratory gases tube assembly, the respiratory gases tube assembly comprising:
a respiratory gases tube configured to transport a breathing gas, the respiratory gases tube comprising a lumen extending from a first end of the respiratory gases tube to a second end of the respiratory gases tube,
an accessory tube, the accessory tube comprising a lumen,
a port connector located at a first end of the respiratory gases tube, the port connector comprising a port extending from the port connector, the port providing for a passageway into a lumen of the port connector, the port comprising:
   a connection mechanism, and
   a port sealing surface,
an accessory tube connector elbow, the connector elbow comprising:
   an accessory tube end configured to connect with the accessory tube, and
   a port end configured to connect with the port,
   a lumen extending from the port end to the accessory tube end,
   an accessory tube connector elbow sealing surface,
a collar, the collar located at the port end of the accessory tube connector elbow,
wherein the collar is configured to engage with the connection mechanism of the port connector to urge the accessory tube connector elbow sealing surface into engagement with the port sealing surface.

In some embodiments, the accessory tube is one or more of:
a pressure line
a sampling tube.

In some embodiments, the port connector is integrally formed with the respiratory gases tube.

In some embodiments, the port connector is provided as a separate connector component which is connectable with the respiratory gases tube.

In some embodiments, the port connector comprises one or more protrusions located within the lumen of the port connector.

In some embodiments, the one or more protrusions extend in a direction along the lumen of the port connector.

In some embodiments, the one or more protrusions are arranged equidistantly around the lumen of the port connector.

In some embodiments, the one or more protrusions comprises three protrusions arranged equidistantly around the lumen of the port connector.

In some embodiments, the connection mechanism is located around at least a part of a perimeter of the port.

In some embodiments, the port connector is or comprises a wye piece.

In some embodiments, the respiratory gases tube is or forms part of an expiratory limb.

In some embodiments, the respiratory gases tube is of forms part of an inspiratory limb.

In some embodiments, the port connector is a pressure port connector, and/or a sampling tube connector, optionally the port connector is an accessory port connector.

In some embodiments, the connection mechanism of the port is one or more of:
a thread
a bayonet.

In some embodiments, the port is substantially cylindrical.

In some embodiments, the port sealing surface is located on an internal surface of the port.

In some embodiments, the port comprises a distal portion located at an accessory tube connector elbow connection end, the distal portion comprising a distal portion tapering surface.

In some embodiments, the distal portion tapering surface tapers towards a proximal end of the port.

In some embodiments, the distal portion provides for alignment of the accessory tube connector elbow and the port when the accessory tube connector elbow is brought into engagement with the port.

In some embodiments, wherein the port comprises a sealing portion, the sealing portion comprising a sealing portion tapering surface.

In some embodiments, the sealing portion tapering surface tapers towards a proximal end of the port.

In some embodiments, the sealing portion comprises the port sealing surface.

In some embodiments, the port comprises a proximal portion, the proximal portion located at a proximal end of the port.

In some embodiments, the port comprises an intermediate portion located between the distal portion and the sealing portion.

In some embodiments, the intermediate portion is configured to maintain alignment of the accessory tube connector elbow and the port, when the accessory tube connector elbow and the port are engaged.

In some embodiments, the port sealing surface is located on an external surface of the port.

In some embodiments, the port extends in a direction away from the lumen of the respiratory gases tube.

In some embodiments, the port extends in a direction substantially perpendicular to the respiratory gases tube.

In some embodiments, the accessory tube connector elbow has an angle between the accessory tube end and the port end of about 10 degrees to about 120 degrees, or about 45 to about 115 degrees, or about 80 to about 100 degrees, or about 90 degrees.

In some embodiments, the accessory tube connector elbow comprises a barbed connection at an accessory tube end configured connect with the accessory tube.

In some embodiments, the accessory tube connector elbow comprises a frictional connection at an accessory tube end configured connect with the accessory tube.

In some embodiments, the accessory tube connector elbow sealing surface is located on an external surface.

In some embodiments, the port end of the accessory tube connector elbow comprises an accessory tube connector elbow tapering surface.

In some embodiments, the accessory tube connector elbow tapering surface comprises the accessory tube connector elbow sealing surface.

In some embodiments, the accessory tube connector elbow sealing surface is located on an internal surface of the accessory tube connector elbow.

In some embodiments, the collar comprises a corresponding connection mechanism on an internal surface of the collar to engage with the connection mechanism of the port connector.

In some embodiments, the collar is rotatable relative to the accessory tube connector elbow.

In some embodiments, the accessory tube connector elbow comprises at least one protrusion extending outwardly from an external surface of the accessory tube connector elbow, the at least one protrusion configured to retain the collar on the accessory tube connector elbow.

In some embodiments, the protrusion extends circumferentially about at least part of the accessory tube connector elbow.

In some embodiments, the protrusion is located near a port end of the accessory tube connector elbow.

In some embodiments, the collar comprises a protrusion, the protrusion configured to engage with the protrusion of the accessory tube connector elbow.

In another aspect there is provided, a respiratory gases tube assembly, the respiratory gases tube assembly comprising:
a respiratory gases tube configured to transport a breathing gas, the respiratory gases tube comprising a lumen extending from a first end of the respiratory gases tube to a second end of the respiratory gases tube,
an accessory tube, the accessory tube comprising a lumen,
a port connector located at a first end of the respiratory gases tube, the port connector comprising a port,
an accessory tube connector elbow, the accessory tube connector elbow comprising:
   an accessory tube end configured connect with the accessory tube and
   a port end configured to connect with the port,
wherein in a first configuration the accessory tube connector elbow is engaged with the port connector but able to rotate relative to the port connector to align the accessory tube with the respiratory gases tube, and
wherein:
   in a second configuration after alignment of the accessory tube and the respiratory gases tube the accessory tube connector elbow is prevented from rotating relative to the port connector to maintain alignment of the accessory tube and the respiratory gases tube, or
   in a second configuration after alignment of the accessory tube and the respiratory gases tube, the resistance to relative rotation of the accessory tube elbow connector and the respiratory gases tube is increased to maintain alignment of the accessory tube and the respiratory gases tube.

In some embodiments, the accessory tube is one or more of:
a pressure line
a sampling tube.

In some embodiments, the accessory tube connector elbow comprises a first connection mechanism and the port connector comprise a second connection mechanism.

In some embodiments, the first connection mechanism and/or the second connection mechanism are threaded connections.

In some embodiments, engaging the first connection mechanism and second connection mechanism urges an accessory tube connector elbow sealing surface connector elbow into engagement with a port sealing surface.

In some embodiments, engaging the first connection mechanism and second connection mechanism, in a first connector configuration, provides for the first configuration.

In some embodiments, engaging the first connection mechanism and second connection mechanism, in a second connector configuration, provides for the second configuration.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7).

The invention is defined by the attached claims.

In this specification, where reference has been made to external sources of information, including patent specifications and other documents, this is generally for the purpose of providing a context for discussing the features of the present invention. Unless stated otherwise, reference to such sources of information is not to be construed, in any jurisdiction, as an admission that such sources of information are prior art or form part of the common general knowledge in the art.

The term "comprising" as used in this specification means "consisting at least in part of". When interpreting statements in this specification which include that term, the features, prefaced by that term in each statement, all need to be present but other features can also be present. Related terms such as "comprise" and "comprised" are to be interpreted in the same manner.

### BRIEF DESCRIPTION OF THE DRAWINGS

The technology will now be described by way of example only and with reference to the drawings in which:
Figures 1A and 1B show a respiratory system.
Figure 2A shows a connector assembly.
Figure 2B shows a respiratory gases tube assembly.
Figure 2C shows a connector assembly.
Figure 2D shows an exploded view of a respiratory gases tube assembly.
Figure 2E and 2F shows a cross section of a respiratory gases tube assembly.
Figure 2G shows a connector assembly.
Figure 2H shows a respiratory gases tube assembly.
Figure 2I shows a connector assembly.
Figure 2J shows an exploded view of a respiratory gases tube assembly.
Figure 2K and 2L shows a cross section of a respiratory gases tube assembly.
Figures 3A to 3D show a connector assembly.
Figures 3E and 3F show a port connector comprising a wye piece.
Figures 4A and 4B show an accessory tube elbow connector.
Figures 4C and 4D show an sensor elbow connector
Figures 5A to 5C show a collar.
Figures 6A and 6B show a respiratory gases tube assembly.

### DETAILED DESCRIPTION

Reference is made in detail to an embodiment, examples of which is illustrated in the accompanying drawings.

During use of respiratory systems, various breathing tubes may be used to form a gases flow path to provide gases to a user (for example as part of a breathing circuit). Properties of the gases may need to be determined at various points in the breathing tube to ensure proper therapy is being delivered to a user.

In some embodiments, the properties of the gases may be determined by one or more sensors. The sensors may be located for example in the breathing circuit, or located elsewhere in the system (for example in an apparatus).

In some embodiments where a sensor is provided, a sensor lead may be required to connect the sensor to a component of the respiratory system (for example a ventilator or respirator or other gases source, or the humidifier). Introducing connector leads or cable increases the number of components to be managed by the user or clinician. The additional connector lead or cable may project into the path of other components or become wound around the respiratory gases tube to which it is connected.

An accessory tube may be used to provide gases and/or a gases pathway from a particular location in the breathing circuit (for example at a location in the respiratory gases tube). The accessory tube may provide a gases pathway back to a component of the respiratory system (for example a ventilator or respirator or other gases source, or the humidifier). The component of the respiratory system may then via the accessory tube measure a property of the gas (for example pressure, or gas concentration.)

It will be appreciated that in some embodiments no gases flow will be provided within the accessory tube, in some embodiments the accessory tube will provide for a flow of gases through the accessory tube.

Introducing an accessory tube to the system will increase the number of components in the system which need to be safely managed by a clinician. Managing the accessory tube may be difficult as it may project into the path of other components of the system or become wound around the respiratory gases tube to which it is connected.

A fixed connection (for example a barbed connection) between the accessory tube and the respiratory gases tube may be provided however this may be difficult for a user to connect with space constraints and does not allow for movement of the accessory tube depending on configuration of the system.

Respiratory gases tubes can be used in breathing circuits or respiratory systems, for example, for delivering and/or removing humidified gases from a patient, such as in obstructive sleep apnea, respiratory humidification (including neonatal patients), and surgical humidification systems including insufflation systems and systems for patients undergoing procedures under general anaesthetic.

This application relates to respiratory gases tube assemblies and/or connectors and/or connector assemblies for use in breathing circuits or respiratory systems. The respiratory gases tubes and connectors may be used for delivering and/or removing humidified gases from a patient, such as in obstructive sleep apnea, respiratory humidification (including neonatal patients), and surgical humidification systems including insufflation systems and systems for patients undergoing procedures under general anaesthetic.

The respiratory gases tube assemblies can be used to deliver gases between two components of a breathing circuit. Respiratory gases tube assemblies can be used to deliver gases between a component and a patient. For example, between a humidifier and a patient.

The respiratory gases tube 10 comprises a lumen 11 that extends from a first end 12 of the respiratory gases tube 10 to a second end 13 of the respiratory gases tube 10.

In one embodiment, the respiratory gases tube 10 is an expiratory limb, or is part of an expiratory limb (for example expiratory tube 172). In another embodiment, the respiratory gases tube 10 is an inspiratory limb or is part of an inspiratory limb (for example expiratory tube 170).

In some embodiments, the respiratory gases tubes can be inspiratory tubes, expiratory tubes, patient interface tubes, supply tubes, dry lines, insufflation tubes, etc.

The respiratory gases tube assemblies may comprise one or more respiratory gases tubes, and one or more connectors.

Tubes for use in respiratory device may be designed to minimise condensation (for example by including insulation and/or heater wires) for example see: PCT Publication No. WO2012164407.

The respiratory gases tubes, connectors and respiratory gases tube assemblies described herein can be provided in one or more respiratory systems, breathing circuits, or kits.

The respiratory gases tubes may be used for delivering and/or removing humidified gases from a patient, such as in obstructive sleep apnea, respiratory humidification (including neonatal patients), and surgical humidification systems including insufflation systems and systems for patients undergoing procedures under general anaesthetic. The respiratory gases tubes can be used to deliver respiratory gases to and/or from a patient as part of a respiratory therapy or treatment. The respiratory gases may be heated and/or humidified prior to delivery to the patient in order to, for example, reduce the likelihood of infection and/or tissue damage.

The respiratory gases tube and accessory tube may be flexible. Flexibility may allow for a user to position and route the tube depending on the particular circumstances of use.

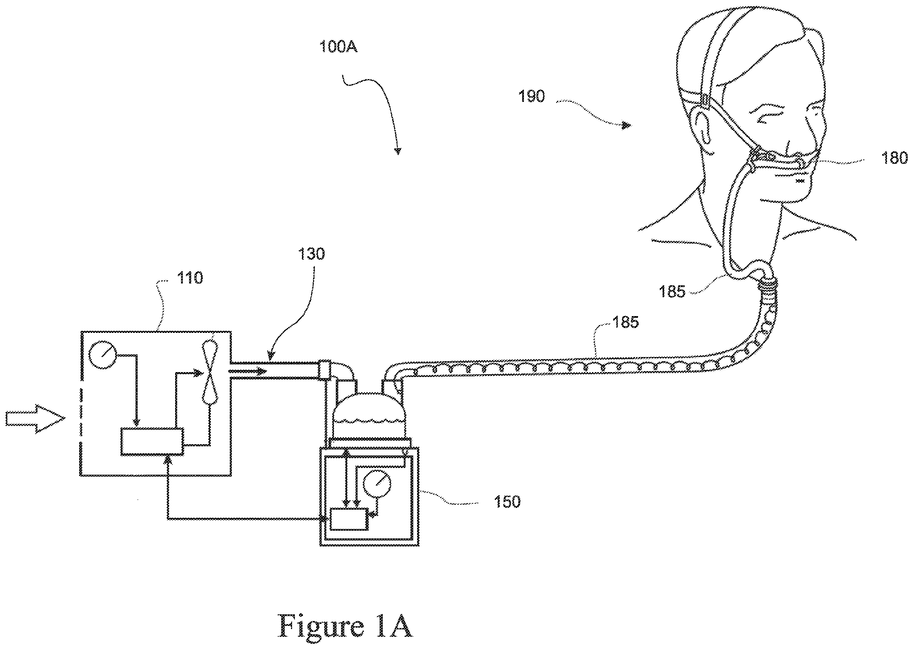

FIG. 1A schematically illustrates an embodiment of respiratory system 100A that can include one or more of the respiratory gases tubes described herein. In the illustrated embodiment, the respiratory system 100A includes a gases source 110 that is either integrated with, or a separate component from, a humidification apparatus 150 (e.g., a humidifier).

The gases source 110 may be for example a ventilator or respirator or other respiratory device configured to deliver gases.

The gases source 110 and/or humidification apparatus 150 supply heated and humidified gases to a patient 190 via a breathing circuit that includes, for example, an inspiratory tube 170 and a patient interface 180.

As used herein, patient interface has a broad meaning and is to be given its ordinary and customary meaning to one of skill in the art, and patient interface also includes, without any limitation, any one or more of a full face mask, a nasal mask, an oral mask, an oral-nasal mask, a nasal pillows mask, nasal cannulas, nasal prongs, a laryngeal mask, or any other suitable coupling between the medical circuit and the airways of the patient.

In some embodiments, another respiratory gases tube, such as a supply tube 130, can be used to transport gases from the gases source 110 to the humidification apparatus 150. Supply tube 130 is sometimes called a "dry" line, as it is positioned in the breathing circuit prior to the "wet" humidifier.

In some embodiments, an additional tube, such as an interface tube 185, can connect between the inspiratory tube 170 and the patient interface 180. It is to be understood that other variations from the system 100A shown may exist. For example, the inspiratory tube 170 may comprise multiple sections to accommodate other equipment such as a water trap, an intermediate connector with one or more sensors, a PCB, and/or a controller.

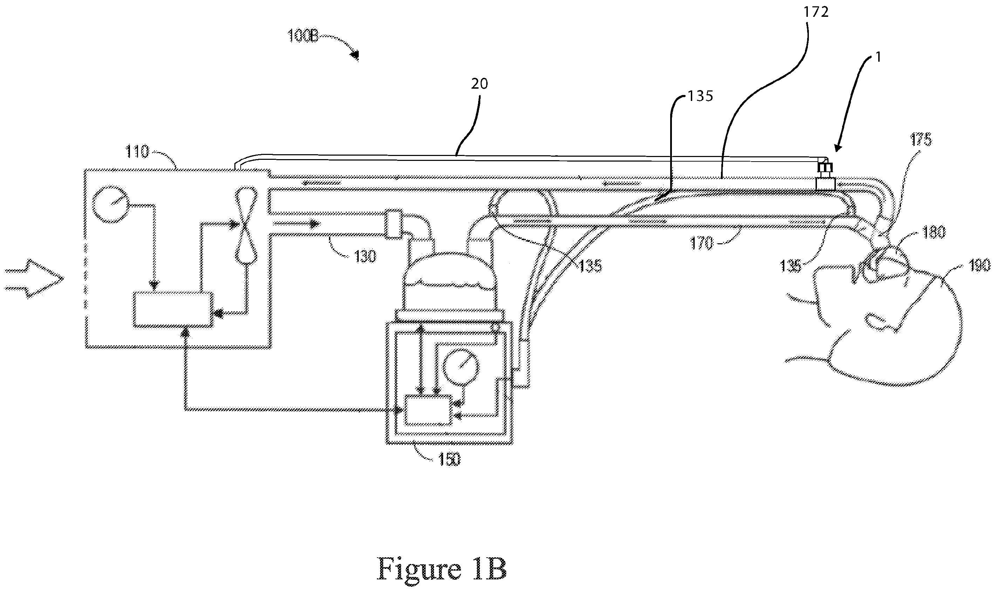

Figure 1B schematically illustrates another embodiment of respiratory system (or breathing circuit) 100B that can include one or more of the respiratory gases tubes described herein. In many respects, the respiratory system 100B can be similar to the respiratory system 100A of FIG. 1. For example, as illustrated, the respiratory system 100B includes a gases source 110 and a humidification apparatus 150 (e.g., a humidifier). The gases source 110 and/or humidification apparatus 150 supplies heated and humidified gases to a patient 190 via a breathing circuit that includes, for example, an inspiratory tube 170 and a patient interface 180. In FIG. 1B, however, the breathing circuit further includes an expiratory tube 172, by which exhaled gases can be transported. In some embodiments, the expiratory tube 172 transports exhaled gases back to the gases source 110 and/or humidification apparatus 150.

In the illustrated embodiment of FIG. 1B, the connector 175 can comprise a y (wye) piece 175 that connects both the inspiratory tube 170 and the expiratory tube 172 to a patient interface component, such as the interface tube 185 (as shown in Fig. 1A), or directly to the patient interface 180 itself. They wye piece 175 is described in more detail below.

Further, the respiratory system 100B can include one or more sensors 135.

For example, a sensor 135 can connect to the inspiratory tube 170 near the patient interface 180 or a sensor 135 can connect to the patient interface 180, among other possible sensor locations. The sensor 135 can be integrated into or connectable to the inspiratory tube 170. In the illustrated embodiment, the system 100B includes two sensors 135, with a first sensor 135 positioned at or nearby to the humidifier chamber outlet end of the inspiratory tube 170, and a second sensor 135 positioned at the patient end of the inspiratory tube 170.

The respiratory system 100B may comprise an accessory tube 20. The accessory tube may provide for a gases pathway between a part of the breathing circuit and a component of the respiratory system 100B.

For example, in Figure 1B an accessory tube 20 is shown as providing a gases pathway between the expiratory tube 172 and the gases source 110.

The accessory tube 20 may be configured to be provided to any part of the breathing circuit (for example the expiratory tube 172, inspiratory limb 170, the patient interface 180, and/or the wye piece 175).

The accessory tube 20 may be configured to be provided to any component of the respiratory system 100B (for example gases source 110, or the humidifier 150).

In some embodiments, the accessory tube 20 may be a pressure line. For example, the accessory tube 20 may be configured to communicate any pressure changes at the location where the accessory tube 20 is connected to the breathing circuit to the component to which the accessory tube 20 is connected.

In some embodiments, the accessory tube 20 may be a sampling tube. For example, the accessory tube 20 may be configured to sample the gases at the location where the accessory tube 20 is connected to the breathing circuit to and provide the gases sample to the component to which it is connected.

The accessory tube 20 may comprise a lumen 21. The lumen 21 provides for a gases pathway.

The respiratory system 100B may comprise a sensor. The sensor may provide for measurement of a property of the gases flow in the breathing circuit. The sensor may be connected to a sensor lead 52 which extends from the sensor to a component of the respiratory system 100B (for example gases source 110, or the humidifier 150). The sensor lead 52 may be located in a similar way to the accessory tube 20 of Figure 1B.

As described in more detail below, the sensor may be provided as part of the sensor connector elbow. In some embodiments, the sensor 53 may be provided at or near the port end 42 of the port connector 30 such that the sensor 53 is provided at least partially in the gases flow path (e.g the lumen) of the port connector 30 when the sensor elbow 60 is coupled to the port connector.

In some embodiments, the sensor connector elbow may provide a sensor port allowing for the insertion of a sensor.

One or more connectors or connector assemblies 1 may be provided in the breathing circuit.

A connector elbow 40, 60 may be used to connect one or more accessories (for example an accessory tube or a sensor lead) with a connector located in the breathing circuit.

The connector elbow may provide a connection from a particular location in the breathing circuit to a component of the respiratory system.

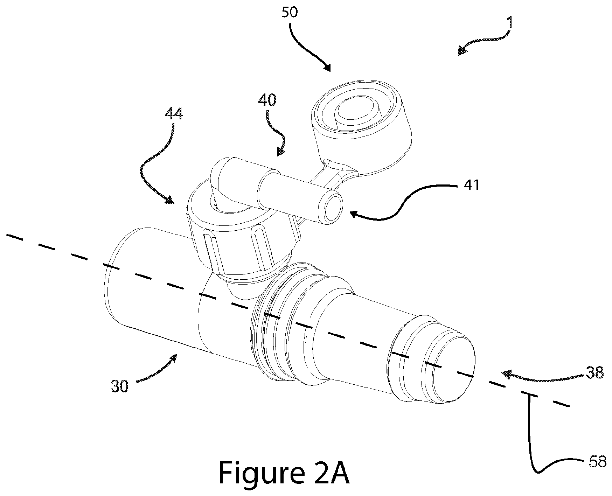

The connector elbow may be an accessory tube connector elbow (for example as shown in Figures 2A-2F.) The accessory tube connector elbow may provide for connection between the breathing circuit and the accessory tube 20.

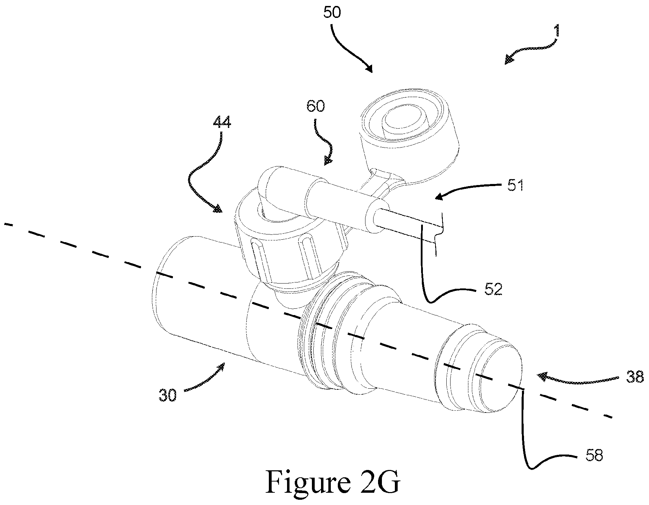

The connector elbow may be a sensor connector elbow (for example as shown in Figures 2G-2L.) The sensor connector elbow may comprise one or more sensors.

Examples below are provided with respect to the accessory tube connector elbow and the sensor connector elbow, it will be appreciated that where context permits, features of these two embodiments may be interchangeable.

The connector assembly 1 may provide for a connection between the breathing circuit and the accessory tube 20 for example as shown in Figure 1B.

As described above it will be appreciated the connector assembly 1 may be located at any location in the breathing circuit (for example the expiratory tube 172, inspiratory limb 170, the patient interface 180, and/or the wye piece 175).

The connector assembly 1 may be connected to or form part of a respiratory gases tube as a respiratory gases tube assembly 1a.

Figures 2A to 6B show a connector assembly 1 which may form part of a respiratory gases tube assembly 1a.

In some embodiments, the connector assembly comprises a port connector 30, and a connector elbow 40, 60. Figures 2A-2L show a connector assembly 1, which has a port connector 30, and a connector elbow 40, 60.

The port connector 30 provides for a connection with a respiratory gases tube 10, and the connector elbow 40, 60.

As shown in Figures 2A-2F, the accessory tube connector elbow 40 provides for a connection between an accessory tube 20 and the port connector 30.

As shown in Figures 2G-2L, the sensor connector elbow 60 may be connected to a sensor lead 52. The sensor lead 52 may be connectable and disconnectable from the sensor connector elbow 60 or integral with the sensor connector elbow 60.

The port connector 30 and the connector elbow 40, 60 may be releasably couplable as described in more detail below.

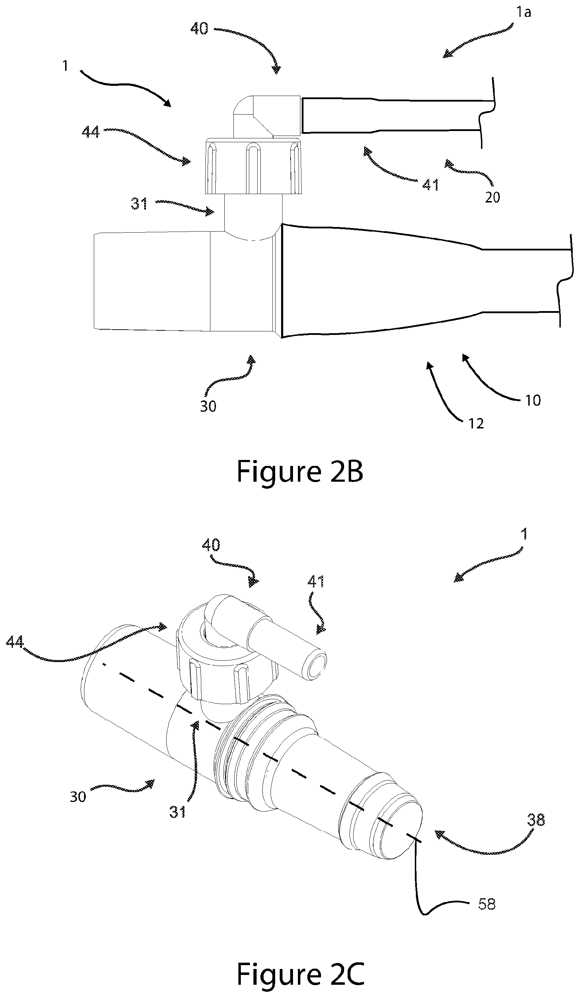

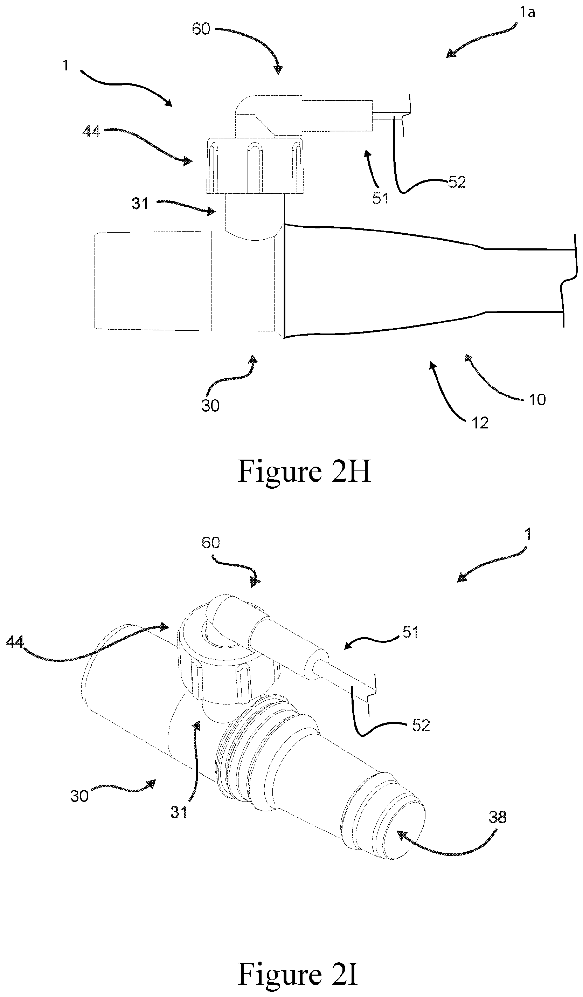

As shown in Figures 2C and 2I the port connector 30 comprises a lumen 38. The lumen 38 provides for a gases pathway from the respiratory gases tube 10 to a further component to which the port connector 30 may be connected.

As shown in Figures 2C and 2G, the port connector 30 may have a central axis 58. In some embodiments the central axis 58 may be of the lumen of the port connector.

The accessory tube connector elbow 40 as shown in Figures 2A-2F comprises a lumen 48. The lumen 48 provides for a gases pathway from the lumen 38 of the port connector 30 to the accessory tube 20.

Figures 2A to 3D show a port connector 30.

The port connector 30 may be connectable to the accessory tube connector elbow 40 and the sensor connector elbow 60.

The port connector 30 is a pressure port connector. In some embodiments the port connector 30 may be a sampling tube connector. In some embodiments the port connector 30 may be an accessory port connector.

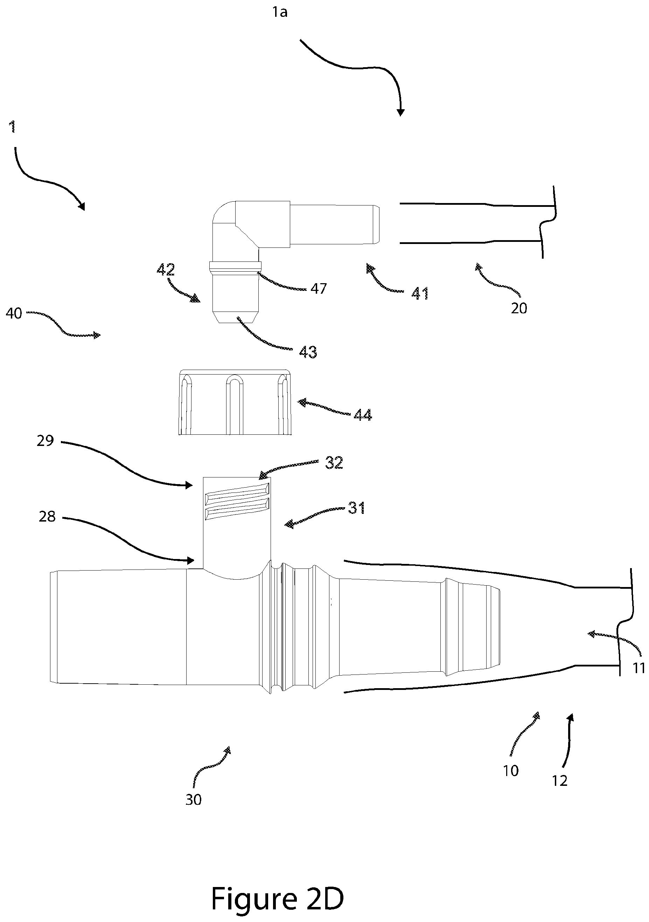

As shown in the connector assembly 1 of Figures 2B and 2D which show the connector assembly 1 as part of a respiratory gases tube assembly 1a, the port connector 30 is located at the first end 12 of the respiratory gases tube 10.

The port connector 30 is provided as a separate connector component which is connectable with the respiratory gases tube 10.

The respiratory gases tube 10 may comprise an intermediate connector configured to provide for a connection between the respiratory gases tube 10 and the port connector 30.

The port connector 30 may be provided at any location on the tube.

The port connector 30 may be integrally formed with the respiratory gases tube 10.

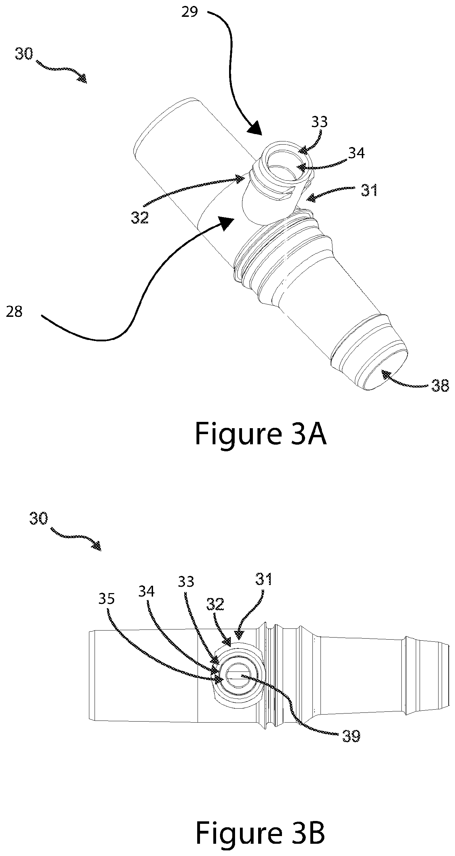

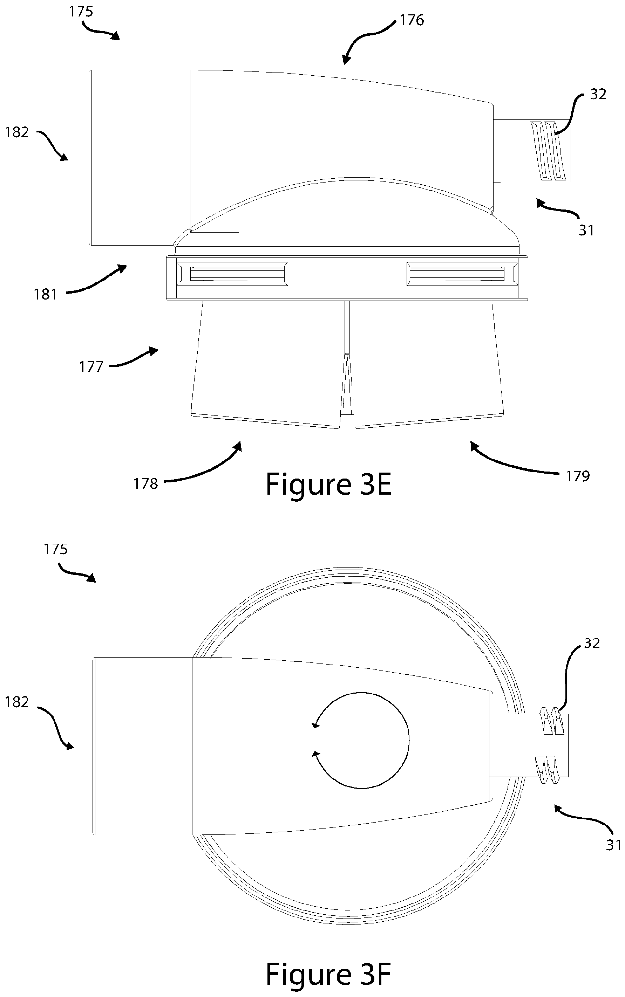

In some embodiments, the port connector 30 is provided on a wye piece 175 as for example shown in Figure 3E and 3F, and described in more detail below.

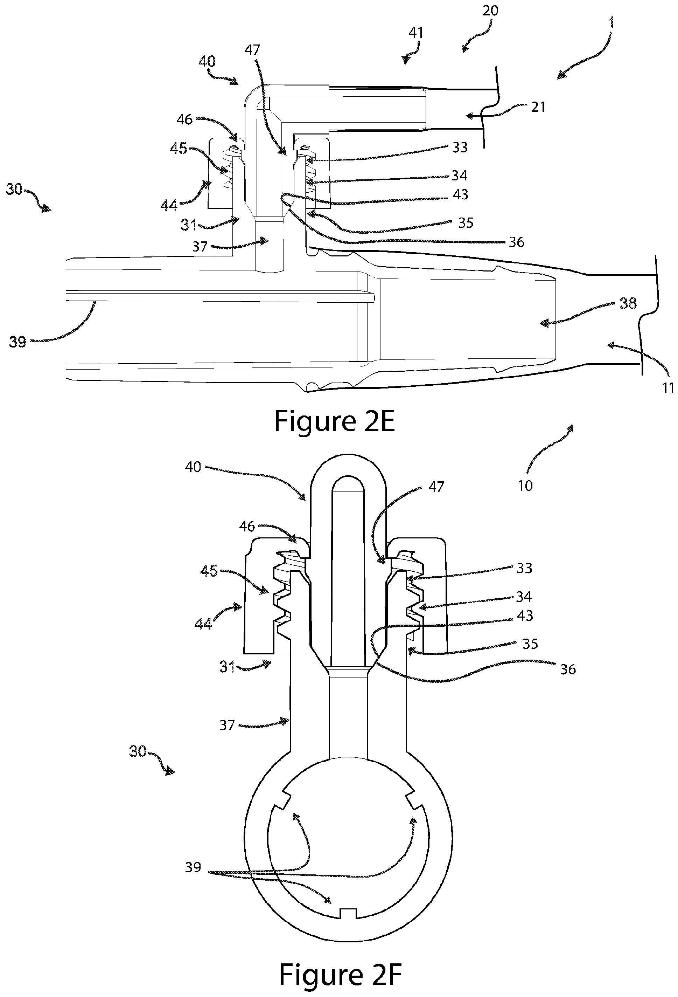

Figure 2D for example shows the port connector 30 having a port 31 extending from the port connector 30. As shown for example in Figure 2E, the port 31 provides a passageway into the lumen 38 of the port connector 30.

The port connector 30 further comprises one or more protrusions 39 located within the lumen 38 of the port connector 30. The protrusions may be configured to prevent internal sealing, so as to prevent misconnection between components. For example, the protrusions may prevent sealing between incompatible components.

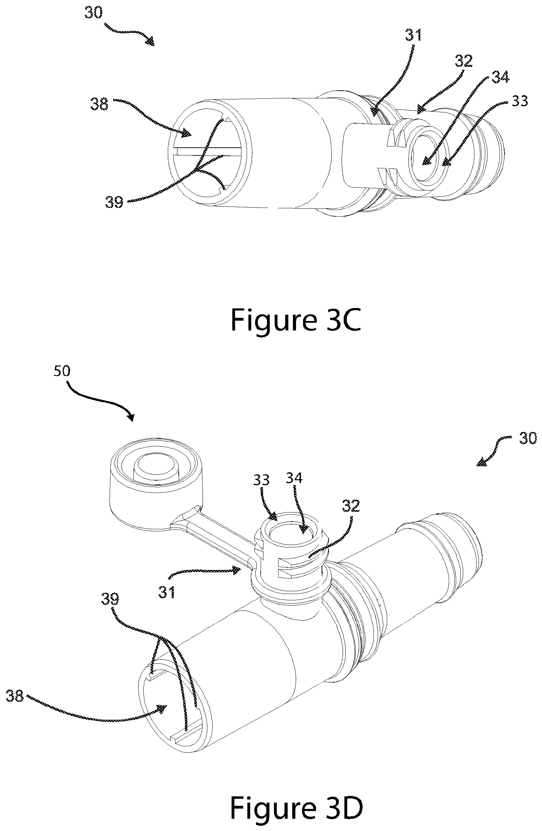

As shown in Figures 2E, 2F and 3D, the one or more protrusions 39 extend along a central axis of the port connector 30.

The protrusions may extend along at least a part, or all of the length of the lumen 38 of the port connector 30.

In some embodiments, the protrusions may be disposed at an angle relative to a length of the port connector 30.

The protrusions may extend from an end of the port connector 30.

The one or more protrusions 39 are arranged equidistantly around the lumen 38 of the port connector 30, as for example shown in Figure 3C.

In one embodiment the one or more protrusions 39 may comprise three protrusions 39 arranged equidistantly around the lumen 38 of the port connector 30.

The port connector 30 may comprise a port 31, as for example shown in Figures 2A-3D.

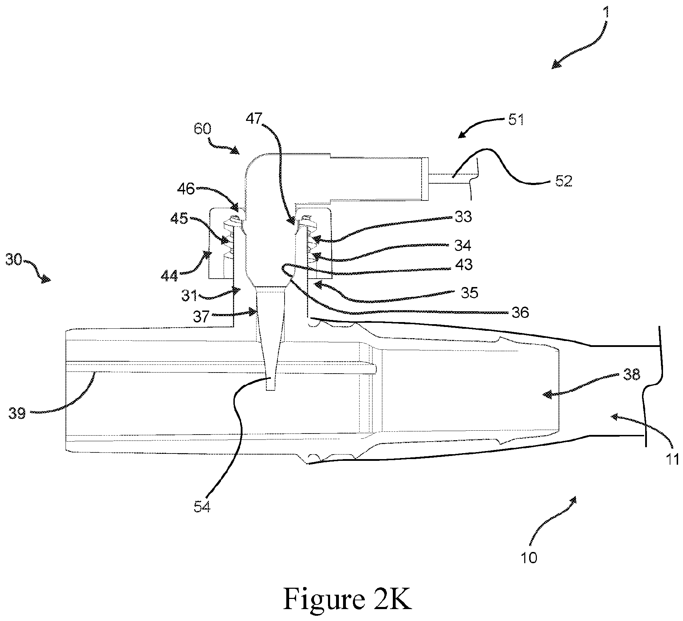

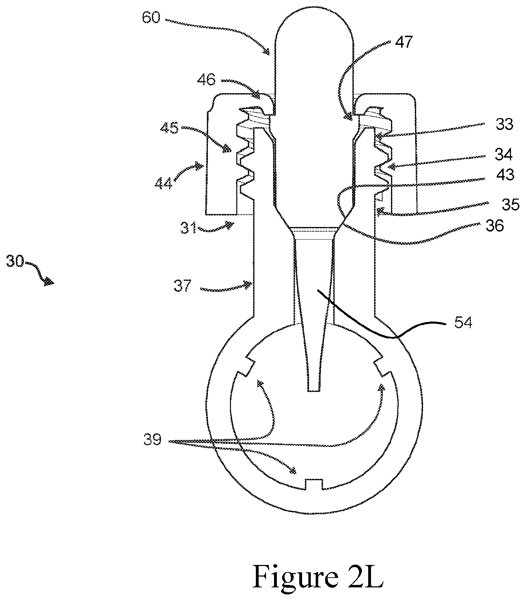

Figures 2E and 2F, 2K and 2L show cross sections of the port connector assembly 1.

As shown in Figure 2E and 2K, the port 31 provided on the port connector 30 may engage the connector elbow 40, 60.

The port 31 may extend in a direction away from the lumen 11 of the respiratory gases tube 10.

In some embodiments the port 31 may extend at an angle that is substantially perpendicular to the respiratory gases tube 10.

The port 31 may extend at an angle relative to the port connector 30. For example the port 31 may extend towards either end of the port connector 30.

The port 31 may be substantially cylindrical in shape, as shown in figures 3A - 3D.

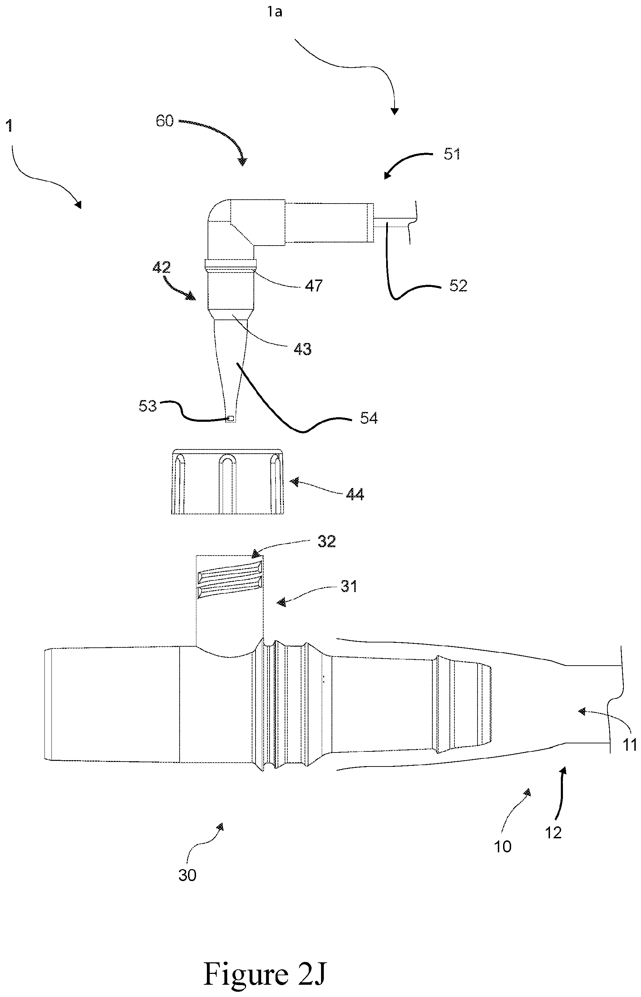

The port 31 has a connection mechanism 32. As shown in Figures 2D, 2J and 3A the connection mechanism 32 is located upon at least a part of a perimeter. In some embodiments the connection mechanism 32 may be located on an external surface of the port 31 (as shown in Figures 2D, 2J and 3A). In some embodiments the connection mechanism 32 may be located on an internal surface of the port 31.

The connection mechanism 32 may be a thread, and/or a bayonet and/or a frictional fit connection.

The port 31 has a distal portion 33. As shown in figure 2E and 2K, the distal portion 33 is located at a distal end 29 of the port 31.

The distal portion 33 has a distal portion tapering surface.

As shown in Figure 2E, 2F, 2K and 2L, the distal portion tapering surface tapers towards a proximal end 28 of the port 31. In some embodiments the distal portion tapering surface may taper towards a distal end 29 of the port 31.

The distal portion tapering surface may transition from a first diameter to a second diameter. The second diameter may be greater than the first diameter. The first diameter may be located further from the distal end 29 of the port 31 than the second diameter. In some embodiments, the second diameter may be located further from the distal end 29 of the port 31 than the first diameter.

The distal portion 33 may provide for alignment of the connector elbow 40, 60 and the port 31 when the connector elbow 40, 60 is brought into engagement with the port 31. This may allow for easier connection between the connector elbow and the port 31 by a user.

The port 31 has an intermediate portion 34. As shown in figure 2E and 2K, the intermediate portion 34 may be provided between the distal portion 33 and the sealing portion 35.

The intermediate portion 34 is configured to maintain alignment of the connector elbow 40, 60 and the port 31, when the connector elbow 40, 60 and the port 31 are engaged. In some embodiments the intermediate portion 34 may provide for a seal with a corresponding surface of the connector elbow 40, 60. In some embodiments, the intermediate portion 34 is configured so as to not seal with a surface of the connector elbow 40, 60 (for example connector elbow sealing surface 43). In some embodiments, the intermediate portion 34 may comprise an intermediate portion tapering surface. The intermediate portion tapering surface may taper towards a proximal end 28 of the port 31. In some embodiments the intermediate portion tapering surface may taper towards a distal end 29 of the port 31.

As illustrated by Figure 2E, 2F, 2K and 2L, the port 31 further comprises a sealing portion 35. For example, as shown in Figure 2E, 2F, 2K and 2L the sealing portion 35 is located at a position immediately adjacent to the intermediate portion 34. In some embodiments, the sealing portion 35 is located further from the distal end 29 of the port 31 than the intermediate portion 34, and/or the distal portion 33.

The sealing portion 35 of the port 31 has a port sealing surface 36. The port sealing surface 36 may be a sealing portion tapering surface.

As shown in Figure 2E, 2F, 2K and 2L, the sealing portion tapering surface tapers towards a proximal end 28 of the port 31. In some embodiments the sealing portion 35 tapering surface may taper towards a distal end 29 of the port 31.

The port sealing surface 36 may transition from a first diameter to a second diameter. The first diameter may be further from the distal end 29 of the port than the second diameter. The second diameter may be greater than the first diameter. In some embodiments, the second diameter may be further from the distal end 29 of the port than the first diameter.

In some embodiments, the port sealing surface 36 may be located on an internal surface of the port 31 (as for example shown in Figures 2E, 2F, 2K and 2L). In another embodiment, the port sealing surface 36 may be located on an external surface of the port 31. It will be appreciated that in embodiments where the port sealing surface 36 is provided on an external surface of the port, the connector elbow 40, 60 may comprise one or more features of the port 31 so as to facilitate connection.

The port sealing surface 36 may be located on an end surface of the port 31 (for example to form a face seal with the connector elbow 40,60).

The port 31 further has an proximal portion 37. As shown in Figures 2E and 2F the proximal portion 37 is located where the port 31 is provided to the port connector 30. The proximal portion 37 enables the flow of gases between the lumen 38 of the port connector 30 and the lumen 48 of the connector elbow 40, 60.

Figures 3E and 3F show a connector assembly 1 where the connector assembly comprises a wye piece 175.

As described above the wye piece 175 may form part of the breathing circuit.

The wye piece 175 may be configured to couple the inspiratory tube 170, expiratory tube 172 and patient interface 180.

The wye piece 175 may comprise a port connector 30 described above.

The wye piece 175 may comprise a first portion 176 and a second portion 177.

The second portion 177 may comprise an inspiratory tube port 178 configured to connect with an inspiratory tube 170 and/or an expiratory tube port 179 configured to connect with an expiratory tube 172.

The first portion 176 may comprise a patient interface port 182 configured to connect with a patient interface 180.

The wye piece 175 may comprise at least one swivel 181 configured to allow for relative rotation of the first portion 176 relative to the second portion 177.

The port 31 as described above may be located on the first portion 176.

The port 31 may extend laterally from the first portion 176 of the wye piece 176.

The port 31 may be located on an opposite side of the first portion 176 than the patient interface port.

In some embodiments, the port 31 may be located on the inspiratory tube port 178, and/or the expiratory tube port 179.

In some embodiments, the port may be provided in a location on the wye piece such that the port 31 is provided substantially perpendicular to the inspiratory limb 170 and/or expiratory limb 172.

In some embodiments, the port 31 may be provided in a location on the wye piece such that the port 31 is provided substantially perpendicular to the inspiratory tube port 178, and/or the expiratory tube port 179.

As described above, the connector assembly 1 may comprise an accessory tube connector elbow 40 (for example as shown in 2A-2F) and/or an sensor connector elbow 60 (for example as shown in 2G-2L).

The connector elbow 40, 60 may comprise an accessory end 41, 51. The accessory end 41, 51 may be configured to be connected with an accessory (for example an accessory tube 20 as an accessory tube end 41 and/or a sensor lead 52 as a sensor lead end 51).

The accessory 20, 52 may be disconnectable and connectable with the accessory end 41, 51.

The accessory 20, 52 may be integrally formed with the connector elbow 40, 60.

As shown in figure 2D the accessory tube connector elbow 40 may comprise an accessory tube end 41 (as an accessory end).

The accessory tube end 41 is configured to connect with the accessory tube 20, as shown in Figures 2B and 2E. In some embodiments, the accessory tube end 41 may comprise a frictional connection mechanism that is configured to connect with the accessory tube 20. In some embodiments, the accessory tube end 41 may comprise a barbed connection mechanism that is configured to connect with the accessory tube 20.

In some embodiments, the accessory tube connector elbow comprises a lumen extending from a port end to an accessory connector end.

In some embodiments, the accessory tube connector elbow is integrally formed with the accessory tube.

The sensor connector elbow 60 may comprise one or more sensors 53.

The one or more sensor 53 may be configured to measure one or more properties of the gases.

The sensor 53 may be one or more of: a pressure sensor, a humidity sensor, a temperature sensor, and/or a flow rate sensor.

The sensor connector elbow 60 may comprise a sensor 53 at or near the port end (described in more detail below) such that the sensor 53 is provided at least partially in the gases flow path of the port connector when the sensor connector elbow 60 is coupled to the port connector.

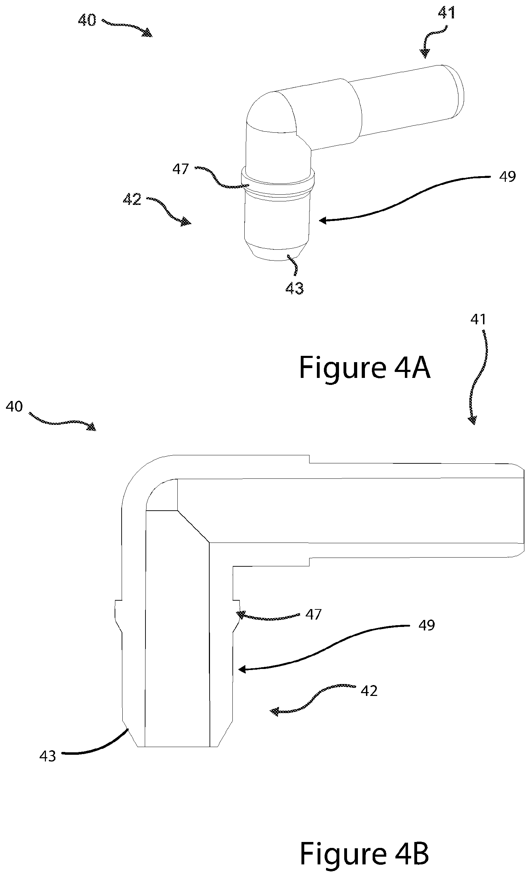

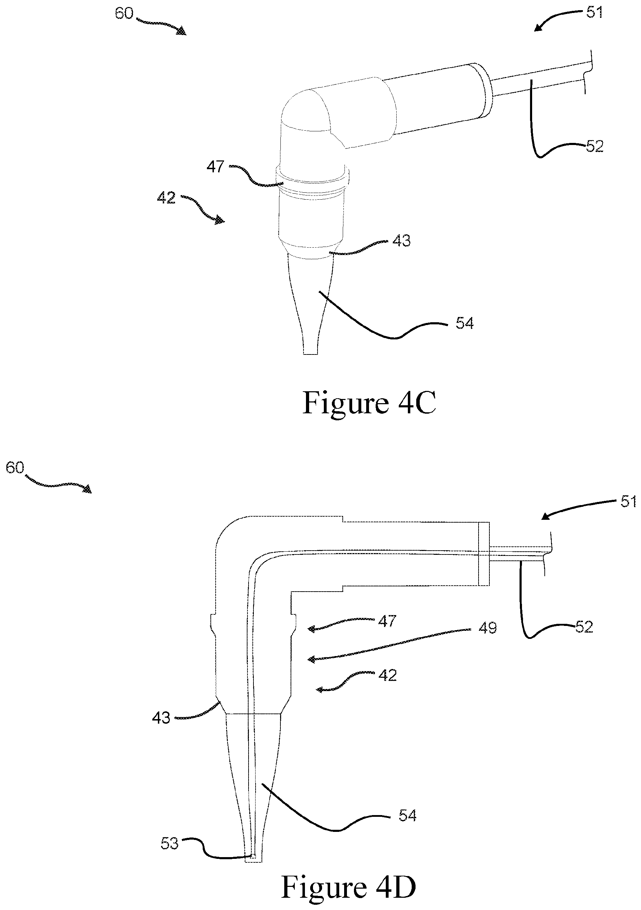

As shown for example in Figures 2J and 4D, the sensor 53 may be located in a probe end 54 of the sensor connector elbow 60. The probe end 54 may extend into the lumen 38 of the port connector 30 when the sensor connector elbow 60 is provided to the port 31 (for example as shown in Figures 2K and 2L).

The sensor lead 52 may extend all the way to the sensor 53 (as shown in Figure 4D) or additional wires may be provided in the sensor connector elbow to connect the sensor 53 with the sensor lead 52.

As shown in figures 2G-2L, 4C and 4D, the sensor connector elbow 60 may comprise an sensor lead end 51 (as an accessory end). The sensor lead end 51 is configured to connect with the sensor lead 52, as shown in figures 2H and 2I.

In some embodiments, the sensor lead end 51 may comprise a frictional connection mechanism that is configured to connect with the sensor lead 52. Additionally or alternatively, the sensor lead 52 may be connected to the lead end 51 by one or more clips.

In some embodiments, the sensor connector elbow 60 is integrally formed with the sensor connector lead 52 at the sensor lead end 51.

In some embodiments, the connector elbow may comprise a lumen extending from the port end to the accessory end and a sensor at the port end such that both an accessory tube and a sensor lead may be provided.

The connector elbow 40, 60 may comprise a port end configured to engage the port of the port connector. For example, the accessory connector elbow 40, 60 has a port end 42, as shown in figure 2D and 2J.

The port end 42 is configured to connect with the port 31.

The connector elbow 40, 60 may have a connector elbow tapering surface, which comprises the connector 40, 60 elbow sealing surface 43. The connector elbow sealing surface 43 may be provided at a port end 42 of the connector elbow 40, 60.

As shown in Figure 2E and 2K, the connector elbow tapering surface tapers towards the port end 42 of the connector elbow, 40, 60. In some embodiments the sealing portion tapering surface may taper away from the port end 42 of the connector elbow 40, 60.

The connector elbow sealing surface 43 may transition from a first diameter to a second diameter. The first diameter may be larger than the second diameter. As shown in Figures 4A to 4D, the first diameter is further away from the port end 42 than the second diameter.

In some embodiments, the first diameter is closer to the port end 42 than the second diameter.

The connector elbow 40, 60 may comprise an intermediate portion 49. The intermediate portion 49 may be configured to engage with the intermediate portion 34 of the port.

In some embodiments, the connector elbow sealing surface 43 may be located on an external surface of the port end 42 (for example as shown in Figures 2E, 2F, 2J, 2K and 2L). In another embodiment the connector elbow sealing surface 43 may be located on an internal surface of the port end 42. It will be appreciated that in embodiments where the connector elbow sealing surface 43 is provided on an external surface of the connector elbow 40, the port 31 may comprise one or more features of the accessory tube connector elbow 40 so as to facilitate connection.

In some embodiments, the connector elbow sealing surface 43 may be located on an end surface of the connector elbow 40, 60 (for example to form a face seal with the port 30).

The angle between a central axis of the accessory tube end 41 or sensor lead end 51 and a central axis of the port end 42 may vary. In one embodiment, the angle may be between about 10 degrees to about 120 degrees. In another embodiment, the angle may be between about 45 to about 115 degrees. In a third embodiment, the angle may be between about 80 to about 100 degrees. In a fourth embodiment, the angle may be about 90 degrees.

In some embodiments, the angle between a central axis of the port 31 and a central axis of the lumen of the port connector 30 and the angle between the accessory tube end 41 or sensor lead end 51 and the port end 42 may sum to about 180 degrees.

The connector elbow 40, 60 further comprises a collar 44.

The collar 44 is located at the port end 42 of the connector elbow 40, 60.

The collar 44 is rotatable relative to the connector elbow 40, 60.

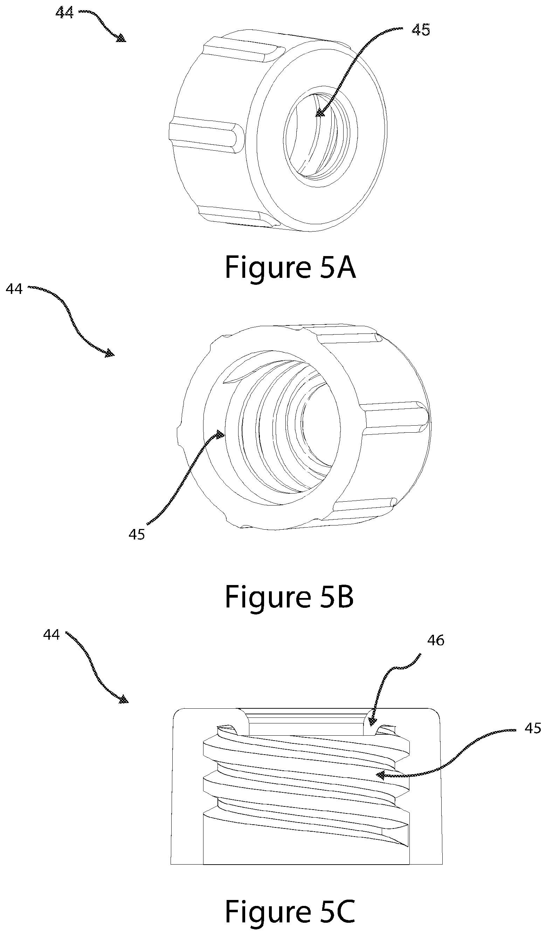

As shown in figures 5A-5C, the collar 44 has a connection mechanism 45 that corresponds to the connection mechanism 32 of the port connector 30. The connection mechanism 45 of the collar 44 is located on an internal surface of the collar 44 to engage with the connection mechanism 32 of the port connector 30.

The collar 44 may comprise at least one protrusion 46. The protrusion 46 of the collar 44 is configured to engage with a protrusion 47 of the connector elbow 40, 60.

The protrusion 46 of the collar 44 may deflect when engaging with the protrusion 47 of the connector elbow 40, 60. The protrusion 46 of the collar 44 may provide a compression force on the protrusion 47 of the connector elbow 40, 60 to force the connector elbow 40, 60 into sealing engagement with the port 31.

The protrusion 47 may be configured to have a tapered surface. The tapered surface may provide for connection between the connector elbow 40, 60 and the collar 44 when the connector elbow 40, 60 is inserted into the collar 40 during assembly.

During assembly of the connector elbow 40, 60 and the collar 44 the protrusion 46 of the collar 44 may be configured to deflect to allow for connection of the connector elbow 40, 60 and the collar 44.

The one or more protrusions 47 of the connector elbow 40, 60 may be configured to retain the collar 44 on the connector elbow 40, 60.

The one or more protrusions 47 on the connector elbow 40, 60 extend outwardly from an external surface of the connector elbow 40, 60, as shown in figures 4A-4D. Figures 4A-4D show that the one or more protrusions 47 extend circumferentially around at least part of the connector elbow 40, 60. The one or more protrusions 47 are located near the port end 42 of the connector elbow 40, 60.

The collar 44 may be configured to engage with the connection mechanism 32 of the port 31 to urge the connector elbow sealing surface 43 into engagement with the port sealing surface 36.

Engaging of the connection mechanism 32 and the collar 44 may provide a force on the connector elbow 40, 60 (via for example the elbow protrusion 47), this force moves the connector elbow 40, 60 into the port 31, so as to engage the port sealing surface 36 and the connector elbow sealing surface 43, and/or provide a compression force between the port sealing surface 36 and the connector elbow sealing surface 43.

The connector assembly 1 may comprise at least one cap 50 (for example as shown in Figures 2A, 2G and 3D.

The cap 50 is configured to engage with the port 31 when the port 31 is not connected with the connector elbow 40, 60.

The cap 50 may be configured to seal with the port 31, optionally in a similar way as the connector elbow 40, 60.

The cap 50 may be retained on the external surface of the port 31, to be retained with the connector assembly 1 when not in use.

As mentioned above it will be appreciated that features of the port 31 and connector elbow 40, 60 may be interchanged. As one example the port 31 may comprise the collar.

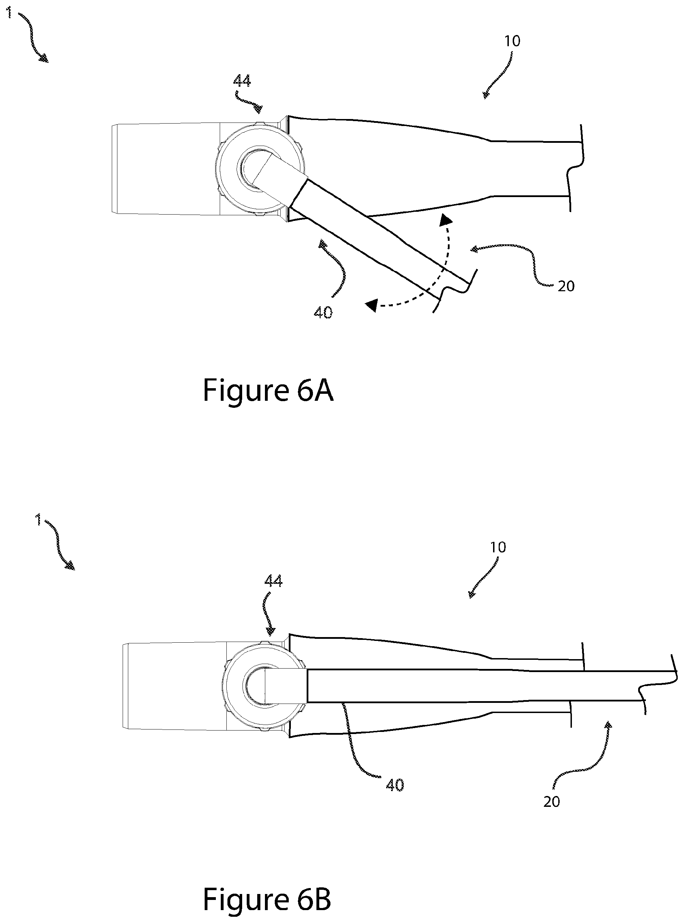

The connector assembly 1 may be configurable to a first configuration for example as shown in Figure 6A.

In a first configuration, the accessory tube connector elbow 40 is engaged with the port connector 30 but able to rotate relative to the port connector 30. This may allow the user to align:
the accessory (for example accessory tube 20, and/or sensor lead 52) with the respiratory gases tube 10 and/or
the accessory (for example accessory tube 20, and/or sensor lead 52) with the accessory end (for example accessory tube end 41 and/or sensor lead end 51) of the connector elbow, and/or
the accessory (for example accessory tube 20, and/or sensor lead 52) with the central axis 58 of the lumen of the port connector, and/or
the accessory end (for example accessory tube end 41 and/or sensor lead end 51) of the connector elbow and the central axis 58 of the lumen of the port connector.

The connector assembly 1 may be configurable to a second configuration for example as shown in Figure 6B. In a second configuration, after alignment as described above with respect to the first configuration, the accessory tube connector elbow 40 is prevented from rotating relative to the port connector 30 to maintain alignment of:
the accessory (for example accessory tube 20, and/or sensor lead 52) and the respiratory gases tube 10, and/or
the accessory (for example accessory tube 20, and/or sensor lead 52) with the accessory end (for example accessory tube end 41 and/or sensor lead end 51) of the connector elbow, and/or
the accessory (for example accessory tube 20, and/or sensor lead 52) and the central axis 58 of the lumen of the port connector, and/or
the accessory end (for example accessory tube end 41 and/or sensor lead end 51) of the connector elbow and the central axis 58 of the lumen of the port connector.

Additionally, or alternatively, in a second configuration after alignment as described above with respect to the first configuration, the resistance to relative rotation of the accessory tube elbow connector 40 and port connector 30 is increased to maintain alignment of:
the accessory (for example accessory tube 20, and/or sensor lead 52) and the respiratory gases tube 10, and/or
the accessory (for example accessory tube 20, and/or sensor lead 52) with the accessory end (for example accessory tube end 41 and/or sensor lead end 51) of the connector elbow, and/or
the accessory (for example accessory tube 20, and/or sensor lead 52) and the central axis 58 of the lumen of the port connector, and/or
the accessory end (for example accessory tube end 41 and/or sensor lead end 51) of the connector elbow and the central axis 58 of the lumen of the port connector.

In some embodiments, the increase in resistance to relative rotation of the accessory tube connector elbow 40 and the port connector 30 of the second configuration may be relative to the resistance to relative rotation of the accessory tube connector elbow 40 and the port connector 30 in the first configuration.

In some embodiments, the increase in resistance to relative rotation of the accessory tube connector elbow 40 and the port connector 30 may still mean that the user can rotate the accessory tube 20 relative the respiratory gases tube 10 (and/or the accessory tube 20 relative to the central axis 58 of the lumen of the port connector) if a sufficiently large force is applied.

It will be appreciated that in Figures 6A and 6B the accessory tube 20 could be replaced with a sensor lead 52.

As described above, the connector elbow 40, 60 may comprise a first connection mechanism (for example connection mechanism 32) and the port connector may comprise a second connection mechanism (for example connection mechanism 45).

As described above, the first connection mechanism and/or the second connection mechanism may be threaded connections.

As described in more detail above engaging the first connection mechanism and second connection mechanism urges an connector elbow sealing surface elbow into engagement with a sealing surface of the port.

In some embodiments, the first connection mechanism and second connection mechanism, in a first connector configuration, provides for the first configuration.

The first connector configuration may be provided where the first connection mechanism and second connection mechanism are partially engaged (for example where the threaded connection is not at a terminal position).

In some embodiments, the first connection mechanism and second connection mechanism, in a second connector configuration, provides for the second configuration.

The second connector configuration may be provided where the first connection mechanism and second connection mechanism are fully engaged (for example where the threaded connection is at a terminal position).

## Claims

1. A connector assembly (1), the connector assembly (1) comprising:
a port connector (30), the port connector (30) being integrally formed with a respiratory gases tube or provided as a separate connector component which is connectable with a respiratory gases tube, the respiratory gases tube (10) configured to transport a breathing gas, the respiratory gases tube comprising a lumen (38) extending from a first end of the respiratory gases tube (10) to a second end of the respiratory gases tube, and the port connector (30) comprising a port (31) extending from the port connector (30), the port (31) providing for a passageway into a lumen (38) of the port connector (30), the port (31) comprising:
a connection mechanism (32), and
a port sealing surface (36),
a connector elbow (40), the connector elbow (40) comprising:
an accessory end (41) configured to connect with an accessory (20),
a port end (42) configured to connect with the port (30),
a connector elbow sealing surface (43), and
a collar (44), the collar (44) located at the port end (42) of the connector elbow (40),
wherein the collar (44) is rotatable relative to the connector elbow (40) and a connection mechanism of the collar (45) is configured to engage with the connection mechanism (32) of the port connector (30) to urge the connector elbow sealing surface (43) into engagement with the port sealing surface (36);
wherein, in a first configuration the connection mechanism of the collar (45) is partially engaged with the connection mechanism of the port (32), and in a second configuration the connection mechanism of the collar (45) and connection mechanism of the port (32) are fully engaged;
wherein in the first configuration the connector elbow (40) is engaged with the port connector (30) but able to rotate relative to the port connector (30) to align a central axis of the accessory end (41) of the connector elbow (40) with a central axis of the lumen (38) of the port connector (30);
wherein in the second configuration, after alignment of the central axis of the accessory end (41) of the connector elbow (40) and the central axis of the lumen (38) of the port connector (30), the resistance to relative rotation of the connector elbow (40) and the port connector (30) is increased to maintain alignment of the accessory and the lumen (38) of the port connector (30).

2. The connector assembly (1) of claim 1, wherein the accessory (20) is integrally formed with the connector elbow (40) or wherein the accessory is disconnectable and connectable with the accessory end (41).

3. The connector assembly (1) of any one of claims 1 to 2, wherein the connector elbow (40) is an accessory tube connector elbow (40), the accessory is an accessory tube (20), and the accessory end (41) is an accessory tube end configured to connect with the accessory tube (20), and optionally wherein the accessory tube connector elbow (40) comprises a lumen (38) extending from the port end (42) to the accessory end (41), or wherein the connector elbow (40) is a sensor connector elbow (40), the accessory is a sensor lead (52), and the accessory end (41) is a sensor lead end configured to connect with the sensor lead (52), and wherein the sensor connector elbow (4) comprises a sensor (53).

4. The connector assembly (1) of claim 3, wherein the connector elbow (4) comprises a barbed and/or frictional connection at the accessory end (41) configured to connect with the accessory tube(20).

5. The connector assembly (1) of any one of claims 1 to 4, wherein the connection mechanism of the port (32) is located around at least a part of a perimeter of the port (31).

6. The connector assembly (1) of any one of claims 1 to 5, wherein the connection mechanism of the port is one or more of:
a thread
a bayonet.

7. The connector assembly (1) of any one of claims 1 to 6, wherein the port (31) comprises a distal portion (33) located at a connector elbow (40) connection end, the distal portion comprising a distal portion tapering surface, and wherein the distal portion tapering surface tapers towards a proximal end of the port (31) and, wherein the distal portion (33) provides for alignment of the connector elbow (4) and the port (31) when the connector elbow (40) is brought into engagement with the port.

8. The connector assembly (1) of any one of claims 1 to 7, wherein the port comprises an intermediate portion (34) located between a distal portion (33) and a sealing portion (35) and, wherein the intermediate portion is configured to maintain alignment of the connector elbow (40) and the port, when the connector elbow (40) and the port are engaged.

9. The connector assembly (1) of any one of claims 1 to 8, wherein the port end (42) of the connector elbow (40) comprises a connector elbow tapering surface and, wherein the connector elbow tapering surface comprises the connector elbow sealing surface (43).

10. The connector assembly (1) of any one of claims 1 to 9, wherein the port (31) extends in a direction away from the lumen (38) of the port connector (30).

11. The connector assembly (1) of any one of claims 1 to 10, wherein engaging the connection mechanism of the collar (45) and the connection mechanism of the port (32) urges the connector elbow (40) sealing surface into engagement with a sealing surface of the port (36).

12. The connector assembly (1) of any one of claims 1 to 11, wherein the connection mechanism of the collar (45) is located on an internal surface of the collar (44) to engage with the connection mechanism of the port connector (32).

13. The connector assembly (1) of any one of claims 1 to 12, wherein the connector elbow (40) comprises at least one protrusion extending outwardly from an external surface of the connector elbow (40), the at least one protrusion configured to retain the collar (44) on the connector elbow (40).

14. The connector assembly (1) of any one of claims 1 to 13, wherein in a second configuration, the resistance to relative rotation of the accessory tube elbow connector (40) and port connector (30) is increased to maintain alignment of the accessory and the respiratory gases tube (10).

15. The connector assembly (1) of any one of claims 1 to 14, wherein the port connector (30) is or comprises a wye piece.

## Patentansprüche

1. Verbindungsanordnung (1), wobei die Verbindungsanordnung (1) umfasst:
einen Anschlussverbinder (30), wobei der Anschlussverbinder (30) integral mit einem Atemgasrohr ausgebildet ist oder als eine separate Verbindungskomponente bereitgestellt ist, die mit einem Atemgasrohr verbindbar ist, wobei das Atemgasrohr (10) zum Transportieren eines Atemgases ausgebildet ist, wobei das Atemgasrohr ein Lumen (38) umfasst, das sich von einem ersten Ende des Atemgasrohrs (10) zu einem zweiten Ende des Atemgasrohrs erstreckt, und wobei der Anschlussverbinder (30) einen Anschluss (31) umfasst, der sich von dem Anschlussverbinder (30) erstreckt, wobei der Anschluss (31) einen Durchgang in ein Lumen (38) des Anschlussverbinders (30) bereitstellt, der Anschluss (31) umfassend:
einen Verbindungsmechanismus (32), und
eine Anschlussdichtfläche (36),
ein Verbindungswinkelstück (40), wobei das Verbindungswinkelstück (40) umfasst:
ein Zubehörende (41), das zum Verbinden mit einem Zubehör (20) ausgebildet ist,
ein Anschlussende (42), das zum Verbinden mit dem Anschluss (30) ausgebildet ist,
eine Verbindungswinkelstückdichtfläche (43), und
einen Kragen (44), wobei sich der Kragen (44) an dem Anschlussende (42) des Verbindungswinkelstücks (40) befindet,
wobei der Kragen (44) relativ zu dem Verbindungswinkelstück (40) drehbar ist und ein Verbindungsmechanismus des Kragens (45) so ausgebildet ist, dass er mit dem Verbindungsmechanismus (32) des Anschlussverbinders (30) in Eingriff kommt, um die Verbindungswinkelstückdichtfläche (43) in Eingriff mit der Anschlussdichtfläche (36) zu drücken;
wobei in einer ersten Konfiguration der Verbindungsmechanismus des Kragens (45) teilweise mit dem Verbindungsmechanismus des Anschlusses (32) in Eingriff steht und in einer zweiten Konfiguration der Verbindungsmechanismus des Kragens (45) und der Verbindungsmechanismus des Anschlusses (32) vollständig in Eingriff stehen;
wobei in der ersten Konfiguration das Verbindungswinkelstück (40) mit dem Anschlussverbinder (30) in Eingriff steht, sich jedoch relativ zu dem Anschlussverbinder (30) drehen kann, um eine Mittelachse des Zubehörendes (41) des Verbindungswinkelstücks (40) mit einer Mittelachse des Lumens (38) des Anschlussverbinders (30) auszurichten;
wobei in der zweiten Konfiguration nach der Ausrichtung der Mittelachse des Zubehörendes (41) des Verbindungswinkelstücks (40) und der Mittelachse des Lumens (38) des Anschlussverbinders (30) der Widerstand gegen eine relative Drehung des Verbindungswinkelstücks (40) und des Anschlussverbinders (30) erhöht wird, um die Ausrichtung des Zubehörs und des Lumens (38) des Anschlussverbinders (30) aufrechtzuerhalten.

2. Verbindungsanordnung (1) nach Anspruch 1, wobei das Zubehör (20) integral mit dem Verbindungswinkelstück (40) ausgebildet ist oder wobei das Zubehör von dem Zubehörende (41) abnehmbar und mit diesem verbindbar ist.

3. Verbindungsanordnung (1) nach einem der Ansprüche 1 bis 2, wobei das Verbindungswinkelstück (40) ein Zubehörrohrverbindungswinkelstück (40) ist, das Zubehör ein Zubehörrohr (20) ist und das Zubehörende (41) ein Zubehörrohrende ist, das zum Verbinden mit dem Zubehörrohr (20) ausgebildet ist, und optional, wobei das Zubehörrohrverbindungswinkelstück (40) ein Lumen (38) umfasst, das sich von dem Anschlussende (42) zu dem Zubehörende (41) erstreckt, oder wobei das Verbindungswinkelstück (40) ein Sensorverbindungswinkelstück (40) ist, das Zubehör eine Sensorleitung (52) ist und das Zubehörende (41) ein Sensorleitungsende ist, das zum Verbinden mit der Sensorleitung (52) ausgebildet ist, und wobei das Sensorverbindungswinkelstück (4) einen Sensor (53) umfasst.

4. Verbindungsanordnung (1) nach Anspruch 3, wobei das Verbindungswinkelstück (4) eine Widerhaken- und/oder Reibungsverbindung an dem Zubehörende (41) umfasst, die zum Verbinden mit dem Zubehörrohr (20) ausgebildet ist.

5. Verbindungsanordnung (1) nach einem der Ansprüche 1 bis 4, wobei sich der Verbindungsmechanismus des Anschlusses (32) um mindestens einen Teil des Umfangs des Anschlusses (31) herum befindet.

6. Verbindungsanordnung (1) nach einem der Ansprüche 1 bis 5, wobei der Verbindungsmechanismus des Anschlusses eines oder mehrere ist von:
einem Gewinde
einem Bajonett.

7. Verbindungsanordnung (1) nach einem der Ansprüche 1 bis 6, wobei der Anschluss (31) einen distalen Abschnitt (33) umfasst, der sich an einem Verbindungsende des Verbindungswinkelstücks (40) befindet, wobei der distale Abschnitt eine sich verjüngende Fläche des distalen Abschnitts umfasst, und wobei sich die sich verjüngende Fläche des distalen Abschnitts zu einem proximalen Ende des Anschlusses (31) hin verjüngt und wobei der distale Abschnitt (33) für die Ausrichtung des Verbindungswinkelstücks (4) und des Anschlusses (31) sorgt, wenn das Verbindungswinkelstück (40) mit dem Anschluss in Eingriff gebracht wird.

8. Verbindungsanordnung (1) nach einem der Ansprüche 1 bis 7, wobei der Anschluss einen Zwischenabschnitt (34) umfasst, der sich zwischen einem distalen Abschnitt (33) und einem Dichtabschnitt (35) befindet, und wobei der Zwischenabschnitt so ausgebildet ist, dass er die Ausrichtung des Verbindungswinkelstücks (40) und des Anschlusses aufrechterhält, wenn das Verbindungswinkelstück (40) und der Anschluss in Eingriff stehen.

9. Verbindungsanordnung (1) nach einem der Ansprüche 1 bis 8, wobei das Anschlussende (42) des Verbindungswinkelstücks (40) eine sich verjüngende Fläche des Verbindungswinkelstücks umfasst und wobei die sich verjüngende Fläche des Verbindungswinkelstücks die Dichtfläche (43) des Verbindungswinkelstücks umfasst.

10. Verbindungsanordnung (1) nach einem der Ansprüche 1 bis 9, wobei sich der Anschluss (31) in einer Richtung von dem Lumen (38) des Anschlussverbinders (30) weg erstreckt.

11. Verbindungsanordnung (1) nach einem der Ansprüche 1 bis 10, wobei das Ineinandergreifen des Verbindungsmechanismus des Kragens (45) und des Verbindungsmechanismus des Anschlusses (32) die Dichtfläche des Verbindungswinkelstücks (40) in Eingriff mit einer Dichtfläche des Anschlusses (36) drückt.

12. Verbindungsanordnung (1) nach einem der Ansprüche 1 bis 11, wobei sich der Verbindungsmechanismus des Kragens (45) an einer Innenfläche des Kragens (44) befindet, um mit dem Verbindungsmechanismus des Anschlussverbinders (32) in Eingriff zu kommen.

13. Verbindungsanordnung (1) nach einem der Ansprüche 1 bis 12, wobei das Verbindungswinkelstück (40) mindestens einen Vorsprung umfasst, der sich von einer Außenfläche des Verbindungswinkelstücks (40) nach außen erstreckt, wobei der mindestens eine Vorsprung so ausgebildet ist, dass er den Kragen (44) auf dem Verbindungswinkelstück (40) hält.

14. Verbindungsanordnung (1) nach einem der Ansprüche 1 bis 13, wobei in einer zweiten Konfiguration der Widerstand gegen eine relative Drehung des Zubehörrohrwinkelstückverbinders (40) und des Anschlussverbinders (30) erhöht ist, um die Ausrichtung des Zubehörs und des Atemgasrohrs (10) aufrechtzuerhalten.

15. Verbindungsanordnung (1) nach einem der Ansprüche 1 bis 14, wobei der Anschlussverbinder (30) ein Y-Stück ist oder umfasst.

## Revendications

1. Ensemble raccord (1), l'ensemble raccord (1) comprenant :
un raccord à orifice (30), le raccord à orifice (30) étant formé d'un seul tenant avec un tube de gaz respiratoires ou fourni en tant que composant de raccord séparé qui peut être raccordé avec un tube de gaz respiratoires, le tube de gaz respiratoires (10) étant configuré pour transporter un gaz de respiration, le tube de gaz respiratoires comprenant une lumière (38) s'étendant d'une première extrémité du tube de gaz respiratoires (10) à une seconde extrémité du tube de gaz respiratoires, et le raccord à orifice (30) comprenant un orifice (31) s'étendant depuis le raccord à orifice (30), l'orifice (31) permettant un passage dans une lumière (38) du raccord à orifice (30), l'orifice (31) comprenant :
un mécanisme de raccordement (32), et
une surface d'étanchéité (36) d'orifice,
un raccord coudé (40), le raccord coudé (40) comprenant :
une extrémité d'accessoire (41) configurée pour se raccorder à un accessoire (20),
une extrémité d'orifice (42) configurée pour se raccorder à l'orifice (30),
une surface d'étanchéité (43) de raccord coudé, et
une bague (44), la bague (44) étant située à l'extrémité d'orifice (42) du raccord coudé (40),
dans lequel la bague (44) peut pivoter par rapport au raccord coudé (40) et un mécanisme de raccordement de la bague (45) est configuré pour s'accoupler avec le mécanisme de raccordement (32) du raccord à orifice (30) afin de pousser la surface d'étanchéité (43) de raccord coudé en accouplement avec la surface d'étanchéité (36) d'orifice ;
dans lequel, dans une première configuration, le mécanisme de raccordement de la bague (45) est partiellement accouplé avec le mécanisme de raccordement (32) de l'orifice, et dans une deuxième configuration, le mécanisme de raccordement de la bague (45) et le mécanisme de raccordement (32) de l'orifice sont pleinement accouplés ;
dans lequel, dans la première configuration, le raccord coudé (40) est accouplé avec le raccord à orifice (30) mais peut pivoter par rapport au raccord à orifice (30) pour aligner un axe central de l'extrémité d'accessoire (41) du raccord coudé (40) avec un axe central de la lumière (38) du raccord à orifice (30) ;
dans lequel dans la deuxième configuration, après l'alignement de l'axe central de l'extrémité d'accessoire (41) du raccord coudé (40) et de l'axe central de la lumière (38) du raccord à orifice (30), la résistance à la rotation relative du raccord coudé (40) et du raccord à orifice (30) est augmentée pour maintenir l'alignement de l'accessoire et de la lumière (38) du raccord à orifice (30).

2. Ensemble raccord (1) selon la revendication 1, dans lequel l'accessoire (20) est formé d'un seul tenant avec le raccord coudé (40) ou dans lequel l'accessoire être désolidarisé de l'extrémité d'accessoire (41) et raccordé avec celle-ci.

3. Ensemble raccord (1) selon l'une quelconque des revendications 1 à 2, dans lequel le raccord coudé (40) est un raccord coudé (40) de tube d'accessoire, l'accessoire est un tube d'accessoire (20), et l'extrémité d'accessoire (41) est une extrémité de tube d'accessoire configurée pour se raccorder au tube d'accessoire (20), et facultativement dans lequel le raccord coudé (40) de tube d'accessoire comprend une lumière (38) s'étendant de l'extrémité d'orifice (42) à l'extrémité d'accessoire (41), ou dans lequel le raccord coudé (40) est un raccord coudé de capteur (40), l'accessoire est un câble de capteur (52), et l'extrémité d'accessoire (41) est une extrémité de câble de capteur configurée pour se raccorder au câble de capteur (52), et dans lequel le raccord coudé (4) de capteur comprend un capteur (53).

4. Ensemble raccord (1) selon la revendication 3, dans lequel le raccord coudé (4) comprend un raccord cannelé et/ou par friction à l'extrémité d'accessoire (41) configuré pour se raccorder au tube d'accessoire (20).

5. Ensemble raccord (1) selon l'une quelconque des revendications 1 à 4, dans lequel le mécanisme de raccordement (32) de l'orifice est situé autour d'au moins une partie d'un périmètre de l'orifice (31).

6. Ensemble raccord (1) selon l'une quelconque des revendications 1 à 5, dans lequel le mécanisme de raccordement de l'orifice est un ou plusieurs parmi :
un filetage
un dispositif à baïonnette.

7. Ensemble raccord (1) selon l'une quelconque des revendications 1 à 6, dans lequel l'orifice (31) comprend une partie distale (33) située à une extrémité de raccordement de raccord coudé (40), la partie distale comprenant une surface de conicité de partie distale, et dans lequel la surface de conicité de partie distale se rétrécit vers une extrémité proximale de l'orifice (31) et, dans lequel la partie distale (33) permet l'alignement du raccord coudé (4) et de l'orifice (31) lorsque le raccord coudé (40) est amené en accouplement avec l'orifice.

8. Ensemble raccord (1) selon l'une quelconque des revendications 1 à 7, dans lequel l'orifice comprend une partie intermédiaire (34) située entre une partie distale (33) et une partie d'étanchéité (35) et dans lequel la partie intermédiaire est configurée pour maintenir l'alignement du raccord coudé (40) et de l'orifice, lorsque le raccord coudé (40) et l'orifice sont accouplés.

9. Ensemble raccord (1) selon l'une quelconque des revendications 1 à 8, dans lequel l'extrémité d'orifice (42) du raccord coudé (40) comprend une surface de conicité de raccord coudé et, dans lequel la surface de conicité de raccord coudé comprend la surface d'étanchéité (43) de raccord coudé.

10. Ensemble raccord (1) selon l'une quelconque des revendications 1 à 9, dans lequel l'orifice (31) s'étend dans une direction s'écartant de la lumière (38) du raccord à orifice (30).

11. Ensemble raccord (1) selon l'une quelconque des revendications 1 à 10, dans lequel l'accouplement du mécanisme de raccordement de la bague (45) et du mécanisme de raccordement (32) de l'orifice pousse la surface d'étanchéité de raccord coudé (40) en accouplement avec une surface d'étanchéité (36) de l'orifice.

12. Ensemble raccord (1) selon l'une quelconque des revendications 1 à 11, dans lequel le mécanisme de raccordement de la bague (45) est situé sur une surface interne de la bague (44) pour s'accoupler avec le mécanisme de raccordement (32) du raccord à orifice.

13. Ensemble raccord (1) selon l'une quelconque des revendications 1 à 12, dans lequel le raccord coudé (40) comprend au moins une saillie s'étendant vers l'extérieur depuis une surface externe du raccord coudé (40), l'au moins une saillie étant configurée pour retenir la bague (44) sur le raccord coudé (40).

14. Ensemble raccord (1) selon l'une quelconque des revendications 1 à 13, dans lequel, dans une deuxième configuration, la résistance à la rotation relative du raccord coudé (40) de tube d'accessoire et du raccord à orifice (30) est augmentée pour maintenir l'alignement de l'accessoire et du tube de gaz respiratoires (10).

15. Ensemble raccord (1) selon l'une quelconque des revendications 1 à 14, dans lequel le raccord à orifice (30) est ou comprend une pièce en Y.
